**Europäisches Patentamt**

**European Patent Office**

**Office européen des brevets**

(19)

(11) Veröffentlichungsnummer: **0 013 029**

**A1**

(12) # EUROPÄISCHE PATENTANMELDUNG

(21) Anmeldenummer: 79105389.5

(22) Anmeldetag: 27.12.79

(51) Int. Cl.³: **G 01 S 15/89**
**G 10 K 11/34, A 61 B 10/00**

(30) Priorität: 02.01.79 US 429
02.01.79 US 430

(43) Veröffentlichungstag der Anmeldung:
09.07.80 Patentblatt 80/14

(84) Benannte Vertragsstaaten:
AT BE CH DE FR GB IT LU NL SE

(71) Anmelder: NEW YORK INSTITUTE OF TECHNOLOGY
Wheatley Road
Old Westbury, New York 11 568(US)

(72) Erfinder: Glenn, William, E., Dr.
650 Royal Plaza
Fort Lauderdale Florida 33 301(US)

(74) Vertreter: Daum, Martin, Dr. et al,
Boehringer Mannheim GmbH Postfach 31 01 20
D-6800 Mannheim 31(DE)

(54) Verfahren und Vorrichtung zur variablen oder wählbaren Verzögerung von, von einer Mehrzahl von Elementen empfangenen Signalen, insbesondere zur Verwendung in einer Vorrichtung zur bildlichen Darstellung eines Körperteils.

(57) Verfahren zur variablen Verzögerung von von einer Mehrzahl von Elementen (1-13) empfangenen Signalen und zur Summierung der Signale, sowie eine Vorrichtung zur Durchführung des Verfahrens, wobei die einzelnen Beiträge der verschiedenen Elemente zum Summensignal gegeneinander verzögert sind und die relativen Verzögerungen zwischen den Signalen verschiedener Elemente sich zeitlich ändern. Ein derartiges Verfahren ist besonders einfach durchzuführen, wenn die Signale von Paaren der Elemente an gegenüberliegende Enden von jeweils einer aus einer Mehrzahl von Verzögerungsketten (210, 220, 230, 240, 250, 260) angelegt werden, die.

Signale an verschiedenen Stufen der Verzögerungsketten durch diesen jeweils zugeordnete Schalteinrichtungen (211, 221,... 261) als Funktion der Zeit abgegriffen werden, so daß die Verzögerungen der von verschiedenen Elementen kommenden Signale sich als Funktion der Zeit ändern, und die Ausgangssignale der Schalteinrichtungen zusammengefaßt werden.

EP 0 013 029 A1

./...

Croydon Printing Company Ltd.

Fig. 5

2340 **0013029**

Verfahren und Vorrichtung zur variablen oder wählbaren
Verzögerung von von einer Mehrzahl von Elementen
empfangenen Signalen

Die Erfindung betrifft ein Verfahren zur variablen Verzögerung von von einer Mehrzahl von Elementen empfangenen Signalen und zur Summierung der Signale, wobei die einzelnen Beiträge der verschiedenen Elemente zum Summensignal gegeneinander verzögert sind und die relativen Verzögerungen zwischen den Signalen verschiedener Elemente sich zeitlich ändern. Weiterhin betrifft die Erfindung eine Vorrichtung zur Durchführung dieses Verfahrens, sowie zur wählbaren Verzögerung von von einer Mehrzahl von Elementen empfangenen Signalen.

In einer ersten Ausführungsform befaßt sich die Erfindung mit einem System zur variablen Signalverzögerung, das im Vergleich zu den bekannten Systemen mit einer geringeren Anzahl von Bauelementen arbeitet und somit Funktionen wie eine dynamische Fokusierung oder Strahlsteuerung in effektiver Weise und bei sparsamer Verwendung von Komponenten erfüllt. Mehrstufige Verzögerungsketten werden zwischen verschiedenen Elementen (z. B. verschiedenen Wandlerelementen oder -abschnitten) "geteilt". Verzögerungsketten und andere Bauteile werden dadurch ökonomisch eingesetzt. In einer zweiten Ausführungsform richtet sich die Erfindung auf ein System zum Verbinden mit wählbarer Verzögerung zwischen einer Mehrzahl von Elementen und einem Eingangs-/Ausgangsanschluß, bei dem die relativen Verzögerungen zwischen dem Eingangs-/Ausgangsanschluß und den einzelnen Elementen vom Bedienungspersonal wählbar sind. Eine einzige Verzögerungskette wird verwendet, um bis zu 3 verschiedene Verzögerungskonfigurationen zu erhalten, die

beispielsweise verwendet werden können, um in einem System zur bildlichen Darstellung drei verschiedene Brennpunkte oder Fokusse zu erhalten. Besonders vorteilhaft anwendbar ist die Erfindung zur Benutzung in einer Vorrichtung zur Ultraschall-Abbildung eines Körpers, bei der Ultraschallenergie in den Körper eingestrahlt wird und die Ultraschallechos aus dem Körper verarbeitet werden.

Die Erfindung betrifft, wie erwähnt, die variable Verzögerung von Signalen und speziell Techniken zur variablen Verzögerung, die für Funktionen wie dynamisches Fokusieren, wählbares Fokusieren oder Strahlsteuern (beam steering) verwendet werden können. Besonders nützlich ist die Erfindung in Ultraschall-Abbildungssystemen.

In der klinischen Diagnose haben in den vergangenen Jahren Ultraschalltechniken zunehmend an Bedeutung gewonnen. Seit einiger Zeit werden derartige Techniken auf den Gebieten der Gynäkologie, Neurologie und Kardiologie benutzt und gewinnen in zunehmendem Maße an Wichtigkeit bei der Darstellung subkutaner Blutgefäße einschließlich der Abbildung relativ kleiner Blutgefäße.

Verschiedenerlei grundlegende Faktoren sind als Ursachen der zunehmenden Benutzung von Ultraschalltechniken anzusehen. Ultraschall unterscheidet sich von anderen Strahlungsformen, insbesondere bezüglich seiner Wechselwirkung mit lebenden Systemen, dadurch, daß er eine mechanische Welle darstellt. Die mit Hilfe von Ultraschall gewonnene Information ist daher in ihrer Natur verschieden von der, die man mit anderen Methoden erhält und es hat sich gezeigt, daß diese Verfahren andere diagnostische Methoden, beispielsweise die Verwendung von Röntgenstrahlen, vorteilhaft ergänzen. Außerdem ist das Risiko von Gewebeschäden nach dem Stand der Erkenntnisse wesentlich geringer als das mit ionisierender Strahlung, beispielsweise Röntgenstrahlung, offenbar verbundene Risiko.

Die Mehrzahl der diagnostischen Verfahren, bei denen Ultraschallenergie benutzt wird, basieren auf der Impuls-echomethode, bei der mit einem geeigneten piezoelektrischen Schallwandler, beispielsweise aus Blei-Zirkonat-Titanat periodisch Impulse von Ultraschallenergie erzeugt werden. Jeder kurze Impuls von Ultraschallenergie wird zu einem engen Strahl fokusiert und in den Körper des Patienten ausgesandt. Dort trifft er auf Grenzflächen zwischen einer Vielzahl verschiedener Körperteile und Strukturen des Körpers. Wenn an einer Grenzfläche eine charakteristische Fehlanpassung der Impedanz (characteristic impedence mismatch) vorliegt, wird ein Teil der Ultraschallenergie an dieser Grenze zu dem Schallwandler zurückreflektiert. Nach Erzeugung eines Impulses wird der Wandler auf "Empfang" geschaltet, so daß er die empfangene reflektierte Energie, d. h. die Echos aus dem Körper, in elektrische Signale zurückverwandelt. Der Zeitpunkt des Eintreffens dieser Echos hängt von der Tiefe der Grenzflächen, auf die der Strahl getroffen ist und von der Ausbreitungsgeschwindigkeit des Ultraschalls ab. Außerdem ist die Amplitude des Echos ein Anzeichen für die Reflektionseigenschaften der Grenzfläche und demzufolge für die Art der charakteristischen Strukturen, die die Grenzfläche bilden.

Die in den empfangenen Echos enthaltene Information kann in verschiedenerlei Weise sinnvoll dargestellt werden. Bei einer gebräuchlichen Methode werden die elektrischen Signale, die den empfangenen Echos entsprechen, verstärkt und an die vertikalen Ablenkplatten eines Kathodenstrahl-Sichtgerätes angelegt. Der Ausgang eines Zeitbasisgenerators wird an die horizontalen Ablenkplatten angelegt. Kontinuierliche Wiederholung des Impulsecho-Prozesses synchron mit den Zeitbasissignalen führt zu einer kontinuierlichen Anzeige, die man als A-Abtastung bezeichnet. Dabei ist die Zeit proportional zur Tiefe der Grenzflächen, die durch vertikale Ablenkungen dargestellt werden. Die Höhe dieser vertikalen Ablenkungen entspricht der Stärke des Echos. Eine andere übliche Form der Darstellung ist die sogenannte B-Abtastung (B-scan), bei der die Echoinformation in einer Form dargestellt wird, die einem gewöhnlichen Fernsehbild ähnlicher ist; d. h. die empfangenen Echosignale werden benutzt, um die Helligkeit des Bildschirms an jedem Punkt der Abtastung zu modulieren. Diese Art der Darstellung ist insbesondere dann sinnvoll, wenn der Ultraschallstrahl eine Abtastbewegung quer zum Körper macht, dergestalt, daß jeweils eine Tiefeninformation (ranging information) eine einzelne entsprechende Zeile oder Abtastlinie des Sichtgeräts ergibt. Entsprechend werden aufeinander folgende in Querrichtung nebeneinander liegende Positionen des Ultraschallstrahls benutzt, um aufeinander folgende Zeilen des Sichtgeräts zu erhalten. Diese Technik liefert ein Querschnittsbild in der Ebene der Abtastung und das resultierende Bildschirmbild kann direkt betrachtet oder photografisch bzw. auf Magnetband gespeichert werden. Die Querabtastung kann man mit Hilfe eines Ultraschallreflektors erreichen, der mechanisch über einen vorgewählten Winkel bewegt wird.

In einem System der beschriebenen Art hat der Schallwandler endliche Größe und der von dem Wandler ausgesandte und/oder empfangene Strahl hat eine endliche Querschnittsfläche. Dadurch wird die mit dem Abbildungssystem erreichbare maximale Auflösung begrenzt. Es ist bekannt, daß der Ultraschallstrahl mit Hilfe einer geeigneten Linse "fokussiert" werden kann, wie sie beispielsweise in der US-PS 3,958,559 beschrieben ist. Eine alternative oder zusätzlich zu verwendende Möglichkeit der Fokussierung besteht darin, den Wandler in Abschnitte oder Elemente zu unterteilen und die verschiedenen Wandlerlemente mit verschieden starker Verzögerung mit der Sender-/Empfängerschaltung zu verbinden. Man kann sich die fokussierende Wirkung eines unterteilten Schallwandlers mit verschiedenen Verzögerungen leicht vorstellen, indem man sich vor Augen hält, daß (für einen flachen Wandler ohne Linse) der Weg des Ultraschalls von oder zu einem gegebenen Fokuspunkt bis zu einem bestimmten aus einer Mehrzahl konzentrischer Wandlerelemente für jedes derartige Wandlerelement verschieden ist. Normalerweise ist der geometrische Weg zwischen dem zentralen Wandlerelement und dem Fokuspunkt am kürzesten und der geometrische Weg zwischen dem Fokuspunkt und dem äußersten Wandlerelement am längsten. Entsprechend hängt der Weg zwischen dem Fokuspunkt und jedem dazwischen liegenden Wandlerelement von dessen Größe und relativer Position in der Reihenfolge der Elemente ab. Folglich würde im allgemeinen Ultraschallenergie, die vom zentralen Element ausgesandt wird, am Fokuspunkt eher eintreffen, als Strahlenergie, die von weiter außen liegenden Wandlerelementen ausgeht. In ähnlicher Weise würde ein Ultraecho, das am Fokuspunkt reflektiert wird, früher am zentralen Wandlerelement eintreffen, als an den äußeren Wandlerlementen. Ein bestimmter Fokus kann daher eingestellt werden, indem man entsprechend längere Verzögerungen (beispeilsweise, aber nicht notwendig, elektronische Verzögerungen) für die mittleren Elemente des Wandlers anwendet als für die äußeren Elemente.

Weiterhin ist bereits bekannt, daß sich die entsprechenden Verzögerungen ändern müssen, wenn sich der betrachtete Fokuspunkt ändert. Dies ist typischerweise der Fall bei Impulsechosystemen, bei denen Information aus einem ausgedehnten Tiefenbereich des Körpers, der mit dem Ultraschallstrahl untersucht werden soll, empfangen wird. Es ist klar, daß bei Benutzung fester Verzögerungen der Strahl nur auf eine bestimmte Brennweite fokussiert ist, d. h. nur auf eine bestimmte Tiefe im Körper. In einer anderen Tiefe liegen andere geometrische Verhältnisse vor und es müssen daher andere Verzögerungen verwendet werden, um eine optimale Fokussierung an jedem Punkt zu erreichen. Man kann sich dies kurz gesagt so vorstellen, daß in entsprechender Weise wie der Fokuspunkt oder Brennpunkt sich tiefer in den Körper bewegt, der Unterschied zwischen dem Ankunftszeitpunkt der Echosignale an den verschiedenen Wandlerelementen immer geringer wird. Entsprechend kann (beim Empfang) eine "dynamische Fokussierung" dadurch erreicht werden, daß man dynamisch die den verschiedenen Wandlerelementen zugeordneten Verzögerungen dergestalt variiert, daß die den mehr zum Zentrum hin gelegenen Elementen zuteil werdenden relativen Verzögerungen abnehmen, wenn der Fokuspunkt sich tiefer in den Körper bewegt. Unglücklicherweise verursacht die Notwendigkeit einer verhältnismäßig großen Anzahl variabler Verzögerungen und einer entsprechenden Schaltung zur Kontrolle dieser Verzögerungen einen solchen Aufwand, daß dynamische Fokussierung für viele Anwendungen als zu aufwendig nicht vertretbar ist. Nachteile der notwendigen elektrischen Schaltung bestehen insbesondere in ihrer unhandlichen Größe, ihren hohen Kosten, ihrer Kompliziertheit und in mangelnder Zuverlässigkeit.

Es ist daher Aufgabe der vorliegenden Erfindung, ein Abbildungsverfahren und eine entsprechende Vorrichtung zur Verfügung zu stellen, die die Technik der dynamischen Fokussierung einschließt, die Nachteile der bekannten Vorrichtungen und Verfahren aber vermeidet. Aufgabengemäß sollen das erfindungsgemäße Verfahren zur variablen Verzögerung und die entsprechende Vorrichtung u.a. zum Strahlsteuern und/oder zu Anwendungen einer variablen Fokussierung verwendet werden können. Systeme mit fester Fokussierung haben Vorteile insoweit, als sie weniger kompliziert und kostspielig sind als dynamische Fokussierungen. Wenn man aber ein Gerät wünscht, das über einen erheblichen Tiefenbereich im Körper arbeiten kann, ist ein System mit festem Brennpunkt oft unangebracht. Andererseits mag in derartigen Fällen eine volle dynamische Fokussierung nicht notwendig sein und zu einer unnötigen Verkomplizierung und Verteuerung führen. Daher besteht eine weitere Aufgabe der vorliegenden Erfindung darin, eine Vorrichtung zur Ultraschalldarstellung zur Verfügung zu stellen, die eine wählbare Fokussierung einschließt, welche im Vergleich zu Festfokus-Techniken bessere Ergebnisse liefert, aber weniger kompliziert ist als die bekannten Techniken zur danymischen Fokussierung.

Diese Aufgaben werden erfindungsgemäß gelöst durch ein Verfahren und eine Vorrichtung der eingangs beschriebenen Art, die durch die in den Ansprüchen angegebenen Merkmale und Maßnahmen charakterisiert sind.

Eine erste Ausführungsform der vorliegenden Erfindung betrifft ein System zur variablen Verzögerung, bei dem weniger Bauelemente, insbesondere auch aktive Bauelemente, verwendet werden, als bei vergleichbaren bekannten Systemen. Das erfindungsgemäße System kann Funktionen wie dynamische Fokussierung oder Strahlsteuern effektiv und unter ökonomischer Verwendung von Komponenten erfüllen. Dabei werden, wie im folgenden dargelegt wird, mehrstufige Verzögerungsketten verwendet, die zwischen verschiedenen Elementen (beispielsweise verschiedenen Wandlerelementen oder -abschnitten) "geteilt" werden. Dadurch und durch andere Eigenschaften ergeben sich erhebliche Vorteile des erfindungsgemäßen Systems gegenüber bekannten Systemen.

Allgemein gesprochen richtet sich eine erste Ausführungsform der vorliegenden Erfindung auf ein System zum Verbinden mit variabler Verzögerung von einer Mehrzahl von Elementen mit einem einzelnen Element. Typischerweise besteht die Mehrzahl von Elementen aus geordneten Elementen oder Abschnitten eines Schallwandlers und das einzelne Element ist ein variabel fokussierter Ausgang. Alternativ kann das einzelne Element aber beispielsweise auch eine Signalquelle sein, die mit variabler Verzögerung an eine Mehrzahl von Elementen angeschlossen ist, wie beispielsweise bei der Technik der elektrischen Strahlsteuerung (electrical beam steering technique). Es ist eine Mehrzahl von Verzögerungsketten vorgesehen sowie Einrichtung , um mit dazwischen geschalteter geeigneter Verstärkung und anderen Einrichtungen, Paare der Elemente mit gegenüberliegenden Enden jeweils einer der Mehrzahl von Verzögerungsketten zu verbinden.

Wenn beispielsweise die Mehrzahl der Elemente n Elemente einschließt, können die als Elemente 1 und Element n numerierten Elemente an gegenüberliegende Enden einer der Verzögerungsketten angeschlossen sein, die Elemente 2 und n-1 können mit gegenüberliegenden Enden einer anderen der Verzögerungsketten verbunden sein und so weiter. Eine Mehrzahl von Schalteinrichtungen (coupling means) ist jeweils der Mehrzahl der Verzögerungsketten zugeordnet, wobei jede der Schalteinrichtungen so gestaltet ist, daß sie eine ausgewählte Verzögerungsstufe ihrer zugeordneten Verzögerungskette mit dem einzelnen Element verbindet. Weiterhin sind Einrichtungen vorgesehen, mit Hilfe derer die gewählten Verzögerungsstufen, die durch die Schalteinrichtungen geschaltet sind, gewechselt werden können.

Bevorzugt sind bei der ersten Ausführungsform der Erfindung die Verzögerungsketten mit einer verschiedenen Anzahl von Verzögerungsstufen ausgestattet und die Schalteinrichtungen sind mit dem einzelnen Element über Verzögerungseinrichtungen verbunden, die dergestalt ausgelegt sind, daß nacheinander größere Verzögerungen zwischen dem einzelnen Element und den Verzögerungsketten mit nacheinander geringerer Zahl von Verzögerungsstufen eingeführt werden. Bei dieser bevorzugten Konstruktion sind die Einrichtungen zum Wechseln der gewählten Verzögerungsstufen, an die die Schalteinrichtungen geschaltet sind, so ausgelegt, daß sie, untereinander synchronisiert, sequentiell durch aufeinander folgende Verzögerungsstufen jeder Verzögerungskette schalten.

Die erste bevorzugte Ausführungsform der vorliegenden Erfindung ist insbesondere, aber nicht notwendigerweise, verwendbar für eine Vorrichtung zur Darstellung eines Körpers, bei der Ultraschallenergie in den Körper eingestrahlt wird. Ein Schallwandler ist zur Umwandlung der aus dem Körper reflektierten Ultraschallenergie in elektrische Signale vorgesehen, wobei der Wandler in eine Reihe geordneter Elemente oder Abschnitte unterteilt ist. Es ist eine Mehrzahl von Verzögerungsketten vorhanden, die jeweils eine verschiedene Anzahl von Verzögerungsstufen haben. Weiter sind Einrichtungen vorgesehen, um die Signale von dem ersten und letzten Element an gegenüberliegende Enden der Verzögerungskette mit der größten Anzahl von Verzögerungsstufen anzulegen, um die Signale von dem zweiten und zweitletzten Element an gegenüberliegenden Enden der Verzögerungskette mit der zweitgrößten Anzahl von Stufen anzulegen und so weiter. Mit der Mehrzahl der Verzögerungsketten ist eine Mehrzahl von Schalteinrichtungen in zugeordneter Weise verbunden, die so ausgelegt sind, daß sie als Funktion der Zeit die Signale von verschiedenen Stufen der Ihnen jeweils zugeordneten Verzögerungsketten abgreifen. Schließlich sind Einrichtungen vorgesehen, um die Ausgänge der Schalteinrichtungen zu einem dem zu formenden Bild entsprechenden Signal zusammenzufassen. Diese Einrichtungen kann man auch als Kopplungseinrichtungen bezeichnen. Besonders bevorzugt hat diese Ausbildungsform der Erfindung Einrichtungen zur Erzeugung einer Mehrzahl synchronisierter Kontrollsignale

sowie Einrichtungen, um die Kontrollsignale an entsprechende Schalteinrichtungen zur Kontrolle der Abgreifpositionen der Schalteinrichtungen anzulegen. Dabei werden die Signale an aufeinanderfolgenden Stufen der Verzögerungsketten sequenziell von den Schalteinrichtungen abgegriffen.

Weiterhin schließen die Einrichtungen zum Zusammenfassen der Signale bei dieser Ausführungsform Verzögerungseinrichtungen ein, die so ausgelegt sind,
daß nacheinander größere feste Verzögerungen für die
Signale angewandt werden, die von Verzögerungsketten
mit nacheinander geringerer Anzahl von Stufen kommen.
Wie im folgenden eingehender beschrieben werden wird,
wird durch diese Technik die Verzögerung durch die
mit einer verschiedenen Stufenzahl ausgerüsteten
Verzögerungsketten mit einer sehr vorteilhaften
Wirkung "kompensiert".

Weiterhin ist es bei der ersten bevorzugten Ausführungsform der Erfindung besonders vorteilhaft, wenn das System
zur variablen Verzögerung (zeitweise) durch das Bedienungspersonal in mehrere verschiedene Zustände mit fester
Verzögerung schaltbar ist. Die entsprechende Anordnung
ist den bereits beschriebenen ähnlich, aber die Mehrzahl
der Schalteinrichtungen muß bei dieser Anordnung nicht
automatisch sequenziell durchgeschaltet werden, um aufeinander folgende Stufen der zugehörigen Verzögerungsketten abzugreifen. Statt dessen ist jede der Schalteinrichtungen so ausgelegt, daß unter Kontrolle des Bedienungspersonals das Signal von der vom Bedieungspersonal gewählten Verzögerungsstufe an die Kopplungseinrichtung angelegt werden kann.
Eine zweite Hauptausführungsform der vorliegenden Erfindung
richtet sich auf ein vergleichsweise kostengünstiges
System zum Herstellen einer Verbindung mit wählbarer Verzögerung zwischen einer Mehrzahl von Elementen und einem
Eingangs-/Ausgangsanschluß (d.h. Eingang- und/oder Ausgangsanschluß), wobei die relativen Verzögerungen zwischen
dem Eingangs-/Ausgangsanschluß und den einzelnen Elementen vom Bedienungspersonal gewählt werden können. Es
wird nur eine einzige Verzögerungskette verwendet, um bis zu drei verschiedene effektive Verzögerungskonfigurationen zu erhalten. Diese können beispielsweise verwendet werden, um in einem System zur Ultraschalldarstellung drei verschiedene Brennpunkte zu erhalten.

Diese zweite Ausführungsform der Erfindung hat bevorzugt eine Verzögerungskette mit einer Mehrzahl von Verzögerungsstufen in einer seriell verbundenen direkt gekoppelten Folge (multiple delay stages in a serially connected string). Es sind Einrichtungen vorgesehen, um die Verzögerungsstufen mit zugeordneten Elementen aus einer Mehrzahl von Elementen zu verbinden. Weiter ist diese Ausführungsform mit einer Schalteinrichtung ausgestattet, um unter Kontrolle des Bedienungspersonals entweder das eine Ende oder das andere Ende der Verzögerungskette mit dem Eingangs-/Ausgangsanschluß zu verbinden. Bevorzugt schließt die Schalteinrichtung weiterhin eine Einrichtung ein, um jedes der Elemente mit dem Eingangs-/Ausgangsanschluß ohne relative Verzögerung zwischen Ihnen zu verbinden. Besonders bevorzugt schließt die Einrichtung zum Verbinden ohne relative Verzögerung einen Teil der Schalteinrichtung ein, der zwischen dem gemeinsamen Pol (common connection) der Verzögerungskette und den Eingangs-/Ausgangsanschluß geschaltet ist. Weiterhin sind bei dieser bevorzugten Ausführungsform im Zusammenhang mit den Schalteinrichtungen Einrichtungen vorgesehen, um eine Abschlußimpedanz an das Ende der Verzögerungskette zu schalten, das nicht an den Eingangs-/Ausgangsanschluß angeschlossen ist.

Die zweite Ausführungsform der vorliegenden Erfindung ist ebenfalls insbesondere in einer Vorrichtung zur Abbildung eines Körpers durch Einstrahlen von Ultraschallenergie in den Körper anwendbar. Eine derartige Vorrichtung schließt üblicherweise einen Impulssender /Empfänger (pulser /receiver) zusätzlich zu einem Schallwandler ein, der eine Anzahl von Elementen zur Abstrahlung von Ultraschallenergie (von dem Impulssender/Empfänger) in den Körper und zur Umwandlung des aus dem Körper reflektierten Ultraschalls aufweist. Diese elektrischen Signale werden an den Impulssender /Empfänger zurückgegeben und dann zu einem zur Darstellung auf einem Sichtgerät geeigneten Bild weiterverarbeitet. Bei Anwendung der zweiten Ausführungsform der vorliegenden Erfindung ist das neuartige wählbare Verzögerungssystem zwischen den Impulssender/Empfänger und die Elemente des Schallwandlers geschaltet.

Im folgenden werden bevorzugte Ausführungsformen der Erfindung anhand der beiliegenden Figuren näher erläutert:

Figur 1 zeigt in schematischer Weise die Anwendung einer erfindungsgemäßen Vorrichtung.

Figur 2 ist eine schematische Darstellung, teilweise als Querschnitt, teilweise als Blockschaltbild, einer Vorrichtung gemäß der ersten bevorzugten Ausführungsform der Erfindung.

Figur 3 ist ein Blockschaltbild einer Vorrichtung zur dynamischen Fokussierung gemäß einer bevorzugten Version der ersten Ausführungsform der Erfindung.

Figur 4 ist ein Blockschaltbild zur Illustration der Abgreif- und Zeittaktschaltung der Ausführungsform nach Figur 3.

Figur 5 zeigt in schematischer Weise, teilweise als Blockschaltbild, teilweise im Querschnitt eine Vorrichtung zur Anwendung der zweiten Ausführungsform der Erfindung.

Figur 6 ist eine schematische Darstellung eines Systems zur wählbaren Fokussierung entsprechend einer bevorzugten Version der zweiten Ausführungsform der Erfindung.

Figur 7 ist ein Diagramm, das die verschiedenen Fokuspunkte, die mit dem System nach der zweiten Ausführungsform der Erfindung erhalten werden können, zeigt.

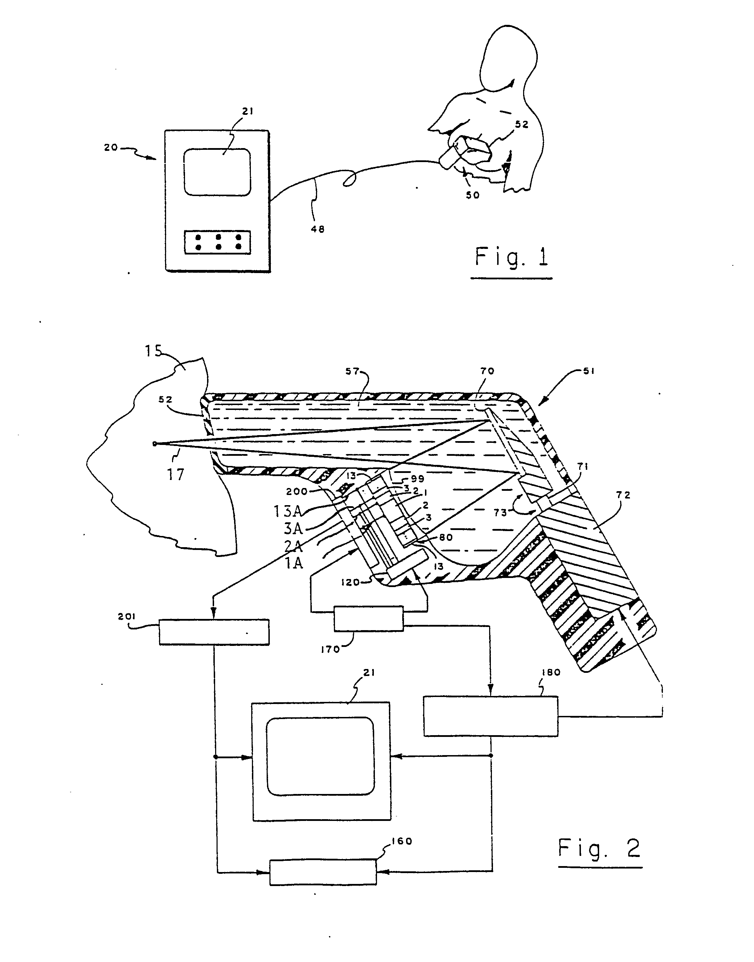

In Figur 1 ist eine Anwendung einer Abtastvorrichtung gemäß der ersten Hauptausführungsform der Erfindung dargestellt. Ein Anzeige- und Kontrollgerät 20 ist mit einem Sichtgerät 21 versehen, das üblicherweise eine Kathodenstrahlröhre ähnlich wie beim Fernsehen sein kann. Weiter weist das Anzeige- und Kontrollgerät 20 ein Bedienungsfeld auf. Auch eine Videoband-Aufzeichnungseinrichtung oder eine geeignete photografische Vorrichtung kann zu dem Anzeige- und Kontrollgerät 20 gehören, um eine Anzeige und Speicherung der Bilder zu ermöglichen. Das Anzeige- und Kontrollgerät wird üblicherweise die Stromversorgung und Teile der Zeittakt- und Verarbeitungsschaltung des im folgenden beschriebenen Systems einschließen. Ein tragbarer Abtastkopf 50 ist über ein Kabel 48 an das Anzeige- und Kontrollgerät 20 angeschlossen. Bei der dargestellten Ausführungsform hat der Abtastkopf einen im wesentlichen zylindrischen Handgriff und ein Abtastfenster 52 bei einem seiner Enden. Im Betrieb der Vorrichtung wird der Abtastkopf 50 von Hand in eine Position gebracht, bei der das Abtastfenster 52 über dem darzustellenden Körperteil angeordnet ist. In Figur 1 ist beispielsweise der Abtastkopf in eine solche Position gebracht, daß ein Querschnittsbild der Brust erhalten wird. Bildliche Darstellungen anderer Körperteile kann man leicht erhalten, indem man den Abtastkopf 50 in die gewünschte Position und Orientierung bringt, wobei die relative Orientierung des Abtastfensters 52 die Winkellage des zu bestimmenden Querschnitts festlegt.

In Figur 2 ist im wesentlichen eine Querschnittsdarstellung eines Teils des Abtastkopfes 50 zusammen mit Diagrammen von Teilen der in dem Abtastkopf und in dem Anzeige- und Kontrollgerät enthaltenen Schaltung wiedergegeben. Ein Gehäuse 51, das aus einem stabilen Kunststoffmaterial hergestellt sein kann, weist ein Abtastfenster 52 an seinem Vorderende auf. Der Abtastkopf 50 wird mit dem Abtastfenster gegen einen Körperteil 15 gehalten.
Das Gehäuse 51 ist mit einer geeigneten Flüssigkeit 57, beispielsweise Wasser gefüllt. Das Abtastfenster 52 ist im wesentlichen flach und kann beispielweise aus Polystyrol oder einem Ployamid bestehen. Eine schallreflektierende Abtasteinrichtung 70, die im folgenden als Schallspiegel bezeichnet wird und in der Zeichnung flach dargestellt ist, aber auch gekrümmt sein kann, um zugleich eine fokussierende Wirkung zu haben, ist in der Nähe des rückwärtigen Endes des Gehäuses 51 angeordnet und im wesentlichen auf das Abtastfenster 52 gerichtet.

Der Schallspiegel 70 ist an einer Achse 71 besfestigt, die eine geeignete Dichtung durchdringt und mit einem elektrischen Motor 72 verbunden ist, der in einer Ausnehmung des Gehäuses angebracht ist. Die Achse 71 wird so angetrieben; daß die gewünschte Hin- und Herbewegung (oscillatory motion) des Schallspiegels 70, angedeutet durch den gekrümmten zweiköpfigen Pfeil 73, erreicht wird.

Ein Ultraschallwandler 80, der mit einer zugeordneten Fokussierlinse 99 versehen sein kann, ist in einem zugehörigen Abteil des Gehäuses 51 untergebracht. Der Wandler ist bezüglich des Schallspiegels 70 in Vorwärtsrichtung in dem Abtastkopf 50 untergebracht, wobei die den Ultraschall abstrahlende Fläche des Wandlers im wesentlichen in dem Abtastkopf 50 rückwärts weist und auf den Schallspiegel 70 gerichtet ist. Wie in der DE-OS 29 11 613 beschrieben wird, ist der Schallwandler 8 so positioniert, daß der von ihm ausgesandte Ultraschallstrahl von dem Schallspiegel 70 reflektiert wird und an dem Wandler 80 vorbei durch das Abtastfenster 52 zurückfällt. Die reflektierende Oberfläche des Abtastspiegels ist bevorzugt aus einem Material hergestellt, das zu einem relativ kleinen kritischen Winkel führt, so daß der nahezu senkrecht auf die Schallspiegeloberfläche auftreffende Strahl den Schallspiegel nicht durchdringt. Durch die beschriebene Anordnung wird das Volumen der Flüssigkeit 57 in dem Abtastkopf 50 effektiv genutzt, da der Strahl 17 an dem Schallwandler vorbei zurückgeworfen wird und somit eine vergleichsweise große Wegstrecke in einem verhältnismäßig kleinen Wasservolumen zurücklegt.

Der Schallwandler 80 ist in eine Mehrzahl von Elemente unterteilt, typischerweise ein kreisförmiges mittleres Element, das von konzentrischen ringförmigen Elementen umgeben ist. Der Schallwandler kann aber auch in der in der DE-OS 29 11 613 beschriebenen Weise eine im wesentlich elliptische Form haben. Insbesondere für andere Anwendungen der Erfindung, beispielsweise das Strahlsteuern, können andere Formgebungen von Schallwandlern, einschließlich linearer Anordnungen, verwendet werden. In Figur 2 sind nur einige der dreizehn Elemente 1,2...13 dargestellt, um die Klarheit der Zeichnung nicht zu beeinträchtigen. Selbstverständlich kann das erfindungsgemäße Prinzip ohne weiteres unabhängig von der Zahl der Elemente verwendet werden. Die Wandlerelemente 1-13 sind mit einer Impulserzeugungsschaltung 120 verbunden, die in bekannter Weise Energieimpulse zum Betrieb des Wandlers 80 liefert. Die Schallwandlerelemente sind außerdem über die Leitungen 1A, 2A...13A mit der neuartigen Schaltung zur dynamischen Fokussierung 200 gemäß der ersten Hauptausführungsform der vorliegenden Erfindung verbunden. Die Schaltung arbeitet vorzugsweise, in der dargestellten Anordnung, während der Empfangsphase (receiving mode) und verarbeitet die empfangenen Echos in der im folgenden beschriebenen Art und Weise. Eine geeignete Vorverstärkung und Verstärkung (in Figur 2 nicht dargestellt) kann in der Schaltung zur dynamischen Fokussierung 200 und in dem Empfänger 201 vorgesehen sein. Der Empfänger 201 kann darüberhinaus dem Fachmann geläufige Verarbeitungselektronik einschließen, die nicht Gegenstand dieser Erfindung ist. Der Ausgang der Schaltung zur dynamischen Fokussierung 200 ist über den Empfänger 201 an das Sichtgerät 21 und das Aufnahmegerät 160 angeschlossen, für das jede geeignete Aufnahme- oder Speichereinrichtung, wie beispielsweise ein Video-Bandgerät verwendet werden kann.

Falls gewünscht kann eine Schaltung zur Regelung des Verstärkungsfaktors vorgesehen sein, die ihrerseits eine interaktive Verstärkungskompensation (interactive gain compensation) einschließen kann, wie sie im einzelnen in der US-PS 4.043.181 beschrieben ist. Die Schaltung zur interaktiven Verstärkungskompensation gleicht die Amplitude der später eintreffenden Signale dahingehend aus, daß die Abschwächung, die sie während der Durchdringung des Körpergewebes erlitten haben und Verluste aufgrund vorheriger Reflektionen kompensiert wird. Die Zeittaktschaltung 170 erzeugt Taktsignale, die der Synchronisation des Systems im Betrieb dienen. Diese Zeittaktsignale sind an die Schaltungen 120 und 200 angelegt um abwechselnd Sendephasen und Empfangsphasen einzuleiten (to alternately energize the transmitting and receiving modes). Außerdem liegen diese Zeittaktsignale an die Schaltung zur Kontrolle der Reflektorbewegung und der Sichtgerät-Ablenkung 180 an, die die Signale erzeugt, die die Hin- und Herbewegung des Abtastspiegels 70 kontrollieren und die das vertikale und horizontale Ablenksignal des Sichtgeräts 21 und des Aufnahmegeräts 160 liefert.

Im wesentlichen arbeitet das System folgendermaßen: Auf ein Trigger-Signal von der Zeittaktschaltung 170 erzeugt die Impulserzeugungsschaltung 120 Impulse,

die die Elemente des Schallwandlers 80 erregen. In der in der Technik bekannten Art und Weise können die Impulse gegeneinander verzögert sein, um eine Fokussierung des Ultraschallstrahles zu bewirken, eine weitere Fokussierung wird durch die Linse 99 erreicht. Die Ultraschallenergie wird von der Oberfläche des Abtastspiegels 70 in den Körper 15 reflektiert, wie es in Figur 2 dargestellt ist, wobei die Linien 17 in etwa die Strahlbegrenzung markieren. Nachdem der Ultraschallstrahl in den Körper ausgesandt ist, leitet die Zeittaktschaltung die Empfangsphase, die man auch als betriebsweise "lauschen" bezeichnen kann, ein, indem die Schaltung 200 eingeschaltet wird. Jetzt dient der Schallwandler 80 dazu, in der Form von Echos aus dem Körper und zurück von dem Abtastspiegel 70 reflektierte Ultraschallenergie in elektrische Signale umzuwandeln. Diese Signale werden nach Verarbeitung durch die Schaltung 200 an das Sichtgerät 21 angelegt. Bei der üblicherweise als "B-Abtastung" bezeichneten Darstellungsweise entspricht ein aus einem Impuls bestehender Abtastvorgang mit entsprechender Erstreckung in die Tiefe des Gewebes einer horizontalen Ablenklinie auf dem Sichtgerät (und resultiert selbstverständlich von der eingestrahlten Energie, die von verschiedenen Grenzschichten in aufeinanderfolgenden Tiefen des Gewebes reflektiert wurde). Die zweite Dimension des gewünschten Querschnittsbildes erhält man durch die langsamere mechanische Ablenkung des Abtastspiegels 70, wobei die Abtastrichtung in der Figur durch den doppelköpfigen Pfeil 73 dargestellt sein soll. Die in diesem Absatz dargestellte Arbeitsweise entspricht im wesentlichen der dem Fachmann

- 20 -

bekannten Technik, wobei die neuartigen Gesichtspunkte der vorliegenden Erfindung unter anderem in der Schaltung zur dynamischen Fokussierung 200 zur Anwendung kommen, die im folgenden beschrieben wird.

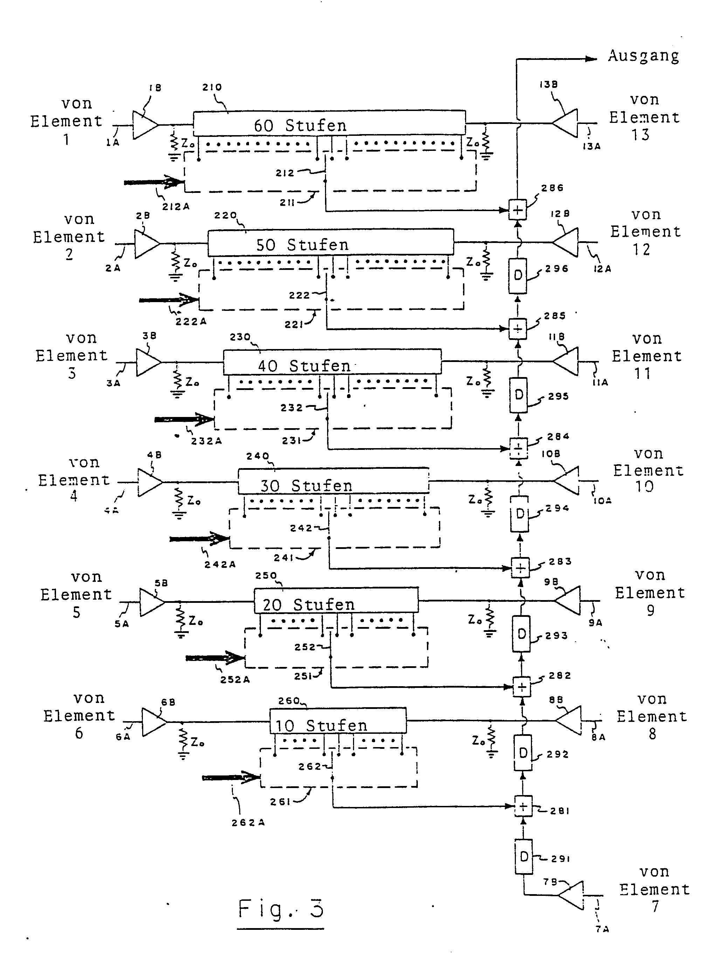

In Figur 3 ist in Form eines Blockschaltbildes eine Schaltung zur dynmaischen Fokussierung 200 nach der vorliegenden Erfindung dargestellt. Wie bereits zuvor dargelegt, wird bei der vorliegenden Ausführungsform zum Zweck einer beispielhaften Erläuterung davon ausgegangen, daß der unterteilte Ultraschallwandler 80 dreizehn Elemente oder Abschnitte hat. Die Elemente, in diesem Falle ein zentrales kreisförmiges Element 1 und 12 konzentrische Ringe 2-13 (Figur 2) sind über die Leitungen 1A-13A an die Schaltung 200 und auch an die Impulserzeugungsschaltung 120 in der zuvor beschriebenen Weise angeschlossen. Selbstverständlich kann die Erfindung auch im Zusammenhang mit anderen Schallwandlertypen und vielerlei anderen räumlichen Anordnungen und Formen des Wandlers verwendet werden. Bei der vorliegenden Ausführungsform sind sechs Verzögerungsketten 210, 220, 230, 240, 250 und 260 vorgesehen, die jeweils auf beiden Seiten mit einer geeigneten Impedanz $Z_o$ abgeschlossen sind. Jede der Verzögerungsketten hat eine verschiedene Anzahl von Stufen, wobei die Verzögerungskette 210 die größte, die Verzögerungskette 220 die zweitgrößte Zahl von Verzögerungsstufen hat und so weiter bis zur Verzögerungskette 260 mit der geringsten Stufenzahl. Die Ausgänge der Schallwandlerelemente 1-13 sind jeweils über zugeordnete Vorverstärker 1B-13B an die Verzögerungsketten angeschlossen. Die Signale vom ersten und letzten Element

des Schallwandlers (d.h. in diesem Fall Element 1 und 13) sind an gegenüberliegende Enden der Verzögerungskette mit der größten Stufenzahl (d.h. Verzögerungskette 210 in diesem Fall) angeschlossen.

Das benachbarte Paar von Wandlerelementen (d.h. Elemente 2 und 12) sind jeweils über Vorverstärker 2B und 12B an gegenüberliegende Enden der Verzögerungskette mit der nächstkleineren Stufenzahl (d.h. Verzögerungskette 220) angeschlossen und so weiter.

In der in Figur 3 dargestellten Ausführungsform sind dann also die Wandlerelemente 3 und 11 über die Vorverstärker 3B und 11B an die gegenüberliegenden Enden der Verzögerungskette 230 angeschlossen, die Wandlerelemente 4 und 10 über die Vorverstärker 4B und 10B an gegenüberliegende Enden der Verzögerungskette 240 angeschlossen, die Wandlerelemente 5 und 9 über Vorverstärker 5B und 9B an gegenüberliegende Enden der Verzögerungskette 250 angeschlossen und schließlich die Wandlerelemente 6 und 8 über die Vorverstärker 6B und 8B an gegenüberliegende Enden der Verzögerungskette 260. Bei der dargestellten Ausführungsform hat die Verzögerungskette 210 sechzig Verzögerungsstufen, die Verzögerungskette 220 fünfzig Verzögerungsstufen, die Verzögerungskette 230 vierzig Verzögerungsstufen, die Verzögerungskette 240 dreißig Verzögerungsstufen, die Verzögerungskette 250 zwanzig Verzögerungsstufen und die Verzögerungskette 260 schließlich zehn Verzögerungsstufen. Jede Verzögerungsstufe hat eine an sie angeschlossene Ausgangsanzapfung. Diese sind in Figur 3 nur bei einigen der Verzögerungsstufen beispielhaft angedeutet.

- 22 -

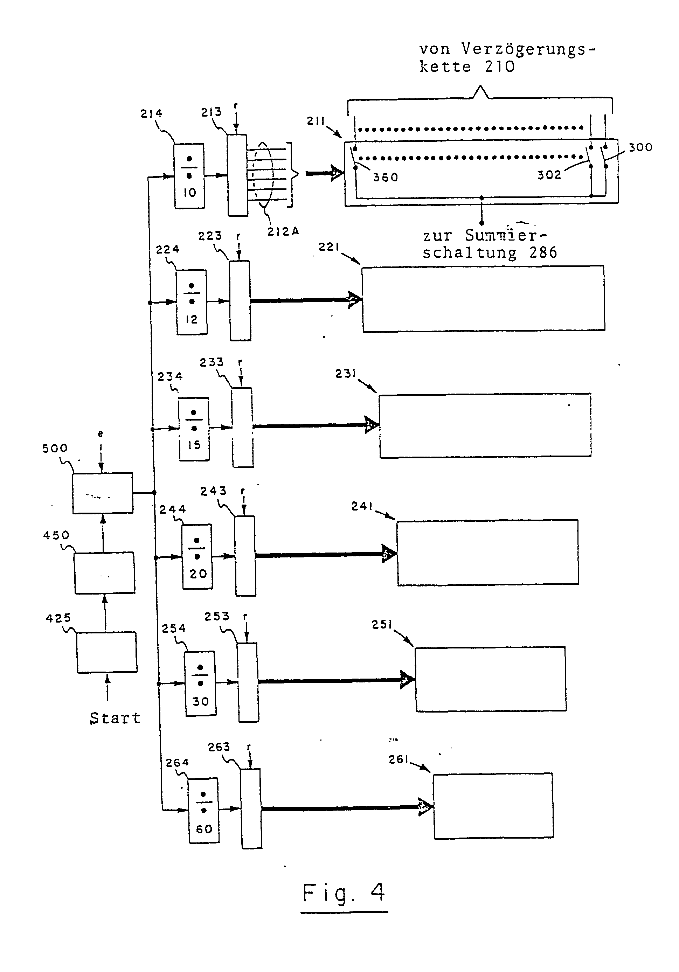

Jede der Verzögerungsketten 210-260 hat eine zugehörige Schalteinrichtung (coupling means) oder Abgreifeinrichtung, die in der Figur mit den Bezugszeichen 211, 221, 231, 241, 251 beziehungsweise 261 bezeichnet sind. Die Abgreif- oder Schalteinrichtungen 211-261, die mehr ins Einzelne gehend im Zusammenhang mit Figur 4 beschrieben werden, sind jeweils so gestaltet, daß sie das Signal an verschiedenen Anzapfungen der zugeordneten Verzögerungsketten abgreifen können. Die momentanen Abgreifpositionen der Schalteinrichtungen sind in der Figur schematisch durch die Kontaktzungen 212, 222, 232, 242, 252 und 262 dargestellt. (Die Darstellung der Schalteinrichtungen als bewegliche Kontaktzungen dient nur der besseren Illustration. Vorzugsweise bestehen die Schalteinrichtungen aus einer Serie insbesondere elektronischer Schalter, die entsprechend angesteuert werden; vgl. Beschreibung zu Figur 4.) Die Positionen der Kontaktzungen werden durch Kontrollsignale bestimmt, die schematisch durch die Pfeile 212A, 222A, 232A, 242A, 252A und 262A dargestellt sind. Wie weiter unten beschrieben werden wird, sind diese Kontrollsignale synchronisiert, beispielsweise dadurch, daß sie von einer gemeinsamen Quelle abgeleitet sind.

Das Ausgangssignal des Schallwandlerelementes 7 und die von den Schalteinrichtungen 211, 221, 231, 241, 251 und 261 abgegriffenen Signale werden kombiniert um ein Signal zu erhalten, das schließlich auf dem Sichtgerät 21 (Figur 1) dargestellt wird. Die Kopplungseinrichtungen (combining means),die zum Zusammenfassen der Signale benutzt werden, schließen Summierungsschaltungen 281, 282, 283, 284, 285 und 286 und Festverzögerungsschaltungen 291, 292, 293, 294, 295 und 296 ein. Diese Summierungs- und Verzögerungsschaltungen sind so zusammengesetzt, daß nacheinander die Signale von Verzögerungsketten mit nacheinander geringerer Stufenzahl größeren, festen Verzögerungen unterliegen. Auf diese Weise wird eine Kompensation für die verschiedene Stufenzahl der verschiedenen Verzögerungsketten erreicht. Im Einzelnen werden die Ausgangssignale der Wandlerelemente, beginnend mit dem Element 7 über die Elemente 6 und 8 bis zu den Elementen 1 und 13 nacheinander durch die Summierungsschaltungen 281, 282...286 zusammengefaßt und dem jeweils zunehmenden Summensignal wird vor jeder neuen Summierung eine feste Verzögerung durch die Festverzögerungsschaltungen 291, 292...296 zugefügt. Anders ausgedrückt ist der Ausgang des Wandlerelements 7 über die Verzögerungsschaltung 291 mit einem Eingang der Summierungsschaltung 281 verbunden, während der andere Eingang der Summierungsschaltung 281 das Signal von der Schalteinrichtung 261 erhält. Der Ausgang der Summierungsschaltung 281 ist dann seinerzeit über die Festverzögerungsschaltung 292 mit einem Eingang der Summierungsschaltung 282 verbunden, deren anderer Eingang das Signal von der Schalteinrichtung 252 erhält und so weiter.

Die grundlegende Arbeitsweise des Systems kann folgendermaßen verstanden werden:

Angenommen jede Verzögerungsstufe jeder Verzögerungskette habe eine charakteristische Verzögerung von einer Verzögerungseinheit und jede der Festverzögerungsschaltungen 291-296 habe eine charakteristische Verzögerung von fünf Verzögerungseinheiten. Wie zuvor

0013029

- 24 -

festgestellt, hat eine beispielhafte Anordnung der vorliegenden Ausführungsform der Erfindung Verzögerungsketten 210, 220...260 mit sechzig, fünfzig ...zehn Verzögerungsstufen. Nimmt man nun an, daß jede der Kontaktzungen 212-262 an der am weitesten rechts liegenden Anzapfung der zugehörigen Verzögerungskette ist, so zeigt die äußerst rechte Spalte der Tabelle I die Anzahl der Verzögerungseinheiten der Signale, die aus jedem der Schallwandlerelemente 1-13 kommen, aufgrund der verschiedenen Verzögerungen in dem System nach Figur 3 unterliegen.

Tabelle I

| Nummer des Schall-wandlerelementes | links | Mitte | rechts |
|:---:|:---:|:---:|:---:|
| | | (Verzögerungseinheiten) | |
| 1 | 0 | 30 | 60 |
| 2 | 5 | 30 | 55 |
| 3 | 10 | 30 | 50 |
| 4 | 15 | 30 | 45 |
| 5 | 20 | 30 | 40 |
| 6 | 25 | 30 | 35 |
| 7 | 30 | 30 | 30 |
| 8 | 35 | 30 | 25 |
| 9 | 40 | 30 | 20 |
| 10 | 45 | 30 | 15 |
| 11 | 50 | 30 | 10 |
| 12 | 55 | 30 | 5 |
| 13 | 60 | 30 | 0 |

Man erkennt leicht, daß in diesem Falle das vom Schallwandlerelement 13 ausgehende Signal keinerlei Verzögerung unterliegt, während das Signal vom Wandlerelement 1 mit sechzig Verzögerungseinheiten verzögert wird, da es alle sechzig Stufen der Verzögerungskette 210 passiert. Diese Resultate sind in der Tabelle dadurch wiedergegeben, daß in der Zeile des Wandlerelementes 13 in der rechten äußeren Spalte eine 0 steht,während in der ersten Zeile, also der dem Wandlerelement 1 zugeordneten, in der gleichen rechten Spalte die Zahl 60 steht. Weiter geht beispielsweise aus der Tabelle hervor, daß das vom Wandlerelement 12 ausgehende Signal fünf Verzögerungseinheiten und das vom Wandlerelement 2 ausgehende fünfundfünfzig Verzögerungseinheiten unterliegt. Dies ist leicht zu verifizieren, wenn man beachtet, daß die Festverzögerungsschaltung 296 fünf Verzögerungseinheiten beiträgt und die Verzögerungskette 220 (bei Stellung der Kontaktzungen ganz rechts) auf Signale aus dem Schallwandlerelement 12 eine Verzögerung von null Verzögerungseinheiten ausübt, während sie das vom Wandlerelement 2 ausgehende Signal um fünfundfünfzig Einheiten verzögert. Die übrigen Angaben in der rechten Spalte der Tabelle I lassen sich in entsprechender Weise nachvollziehen, d.h. indem man die entsprechende Anzahl Verzögerungseinheiten von den Festverzögerungsschaltungen und der zugeordneten Verzögerungskette addiert. So wird beispielsweise das von dem Schallwandlerelement 7 ausgehende Signal einer Verzögerung um dreißig Verzögerungseinheiten unterliegen (in allen Fällen), da es sechs Festverzögerungsstufen 291-296 passiert.

In Tabelle I ist auch angegeben, wieviel Verzögerungs-einheiten die von jedem der Schallwandlerelemente ausgehenden Signale unterliegen, wenn die Kontaktzungen 212, 222...262 alle in ihrer ganz linken Stellung sind. Die entsprechenden Zahlen finden sich in der linken Spalte der Tabelle I unter dem Wort links. Die aufgeführten Verzögerungen haben die umgekehrte Reihenfolge derjenigen in der rechten Spalte von Tabelle I. So unterliegt das Signal aus dem Wandlerelement 1 jetzt der Verzögerung 0, während das vom Wandlerelement 13 kommende Signal jetzt mit sechzig Verzögerungseinheiten verzögert wird. Die übrigen aufgelisteten Verzögerungen sind wiederum in entsprechender Weise zu erhalten.

In der mittleren Spalte der Tabelle I ist die Anzahl der Verzögerungseinheiten angegeben, denen Signale aus jedem der Wandlerelemente unterliegen, wenn die Kontaktzungen alle in der mittleren Stellung der zugeordneten Verzögerungsketten 210, 220...260 (d.h. gleich weit entfernt von den Endanzapfungen) sind. Wie man sieht, erfährt das von jedem Schallwandlerelement ausgehende Signal in diesem Fall eine Verzögerung von dreißig Einheiten. So passieren beispielsweise die Signale aus den Wandlerelementen 1 und 13 jeweils die Hälfte der insgesamt sechzig Stufen der Verzögerungsleitung 210 (d.h. je dreißig Ver-zögerungseinheiten). Die aus den Wandlerelementen 2 und 12 hervorgehenden Signale erfahren jeweils eine Verzögerung von fünfundzwanzig Einheiten, indem sie jeweils durch eine Hälfte der Verzögerungskette 220 laufen und unterliegen darüberhinaus einer zu-

- 27 -

sätzlichen Verzögerung von fünf Verzögerungseinheiten aufgrund der Festverzögerungsschaltung 296, so daß sich wiederum für jedes dieser beiden Signale eine Gesamtverzögerung von dreißig Einheiten ergibt.

Durch eine entsprechende Analyse läßt sich ermitteln, daß die Signale von jedem der Wandlerelemente in diesem Fall jeweils mit dreißig Einheiten verzögert werden, entsprechend der mittleren Spalte der Tabelle I. Die Arbeitsweise des erfindungsgemäßen Verfahrens zur variablen oder dynamischen Fokussierung kann nun leicht verstanden werden, wenn man sich vorstellt, was geschieht, wenn die Kontaktzungen 212, 222...262 zugleich über die Anzapfungen der zugehörigen Verzögerungsketten 210, 220...260 streichen. Wenn die Kontaktzungen in der Mitte jeder Verzögerungskette sind, und somit die Signale aus allen Wandlerelementen der gleichen Verzögerung unterliegen (im vorliegenden Beispiel dreißig Einheiten) ist das System auf den geometrischen Brennpunkt des Schallwandlers fokussiert. Wenn die Kontaktzungen an der am weitesten rechts liegenden Anzapfung der zugeordneten Verzögerungsketten sind, werden die größten Verzögerungen den Signalen aus den Wandlerelementen mit den kleinen Nummern (d.h. bei der vorliegenden Ausführungsform den weiter innen liegenden Wandlerelementen) zugefügt und als Resultat ist das Empfangssystem auf einen "nahen" Brennpunkt fokussiert, der näher beim Schallwandler liegt, als der geometrische Brennpunkt. Dabei kompensiert die größere Verzögerung für die weiter innen liegenden Wandlerelemente den verhältnismäßig kürzeren Laufweg vom Fokuspunkt zu diesen Wandlerelementen und daraus resultiert die angesprochene effektive Fokussierung auf einen "nahen" Brennpunkt. Das entgegengesetzte Resultat stellt sich ein, wenn sich die Kontaktzungen an den am meisten links liegenden Anzapfungen der zugehörigen Verzögerungsketten befinden. Dies führt zu einem "fernen" Fokuspunkt, der vom Schallwandler weiter entfernt ist als der geometrische Fokus. Für zwischen den beschriebenen liegende Positionen der Kontaktzungen liegt der Fokus auf entsprechenden Zwischenpositionen.

Im Betrieb gibt auf einen Befehl der Zweitaktschaltung 170 die Impulserzeugungsschaltung 120 einen Energieimpuls an den Wandler 80 und der Ultraschallstrahl wird in den zu untersuchenden Körper abgesandt. (Normalerweise, aber nicht notwendigerweise, ist das System zur dynamischen Fokussierung während der Sendephase nicht in Betrieb und der Strahl daher auf den geometrischen Brennpunkt des Systems fokusiert.) Zu einem bestimmten Zeitpunkt nach Aussendung des Impulses, wobei dieser Zeitpunkt eine Funktion der Entfernung des gewünschten nahen Fokuspunktes und der Ultraschallgeschwindigkeit in den durchlaufenden Medien ist, wird die Funktion des Untersystems nach Figur 3 eingeleitet, wobei die Kontaktzungen 212, 222...262 sich in ihrer äußersten rechten Stellung befinden. Die Kontaktzungen laufen dann simultan nach links, wobei die Laufzeit so eingestellt ist, daß sie im wesentlichen übereinstimmt mit der erwarteten Laufzeit des Ultraschalls im zu untersuchenden Körper zwischen dem nahen und fernern Fokuspunkt. Demzufolge läuft der vom System bestimmte Fokus im wesentlichen der Strahlposition nach (substantially tracks the beam position),so daß die von einer Grenzfläche im interessierenden Tiefenbereich zurückkommenden Echos automatisch scharf fokussiert sind. Dieses allgemeine Prinzip der dynamischen Fokussierung ist bekannt, aber die beispielhaft anhand des in Figur 3 dargestellten Systems beschriebene Technik, bei der Verzögerungseinrichtungen in effektiver Weise "geteilt" werden, weist gegenüber dem bekannten Stand der Technik erhebliche Vorteile auf, da die notwendige Zahl an Komponenten vermindert ist und damit das normalerweise für eine variable Fokussierung notwendige komplizierte System erheblich vereinfacht ist.

Figur 4 zeigt ein Blockschaltbild der Zeittakterzeugungsschaltung, wie sie zur Kontrolle der Signale 212A,222A....262A nach Figur 3 verwendet werden kann. Außerdem zeigt sie eine Ausführungsform der Schalt- oder Abgreifeinrichtungen 211, 221...262, wobei nur eine dieser Schaltungen aus Gründen der Übersichtlichkeit mehr ins einzelne gehend dargestellt ist. Jede Schalteinrichtung schließt eine Mehrzahl von über Adressen kontrollierten Schaltern (address controlled switches) ein, wie z.B. die Schalter 300, 301...360 der Schalteinrichtung 211. Ein Anschluß von jedem dieser Schalter ist an eine Anzapfung der zugehörigen Verzögerungskette angeschlossen (Figur 3). Die jeweils anderen Anschlüsse der Schalter sind zusammengeschaltet und bilden einen gemeinsamen Ausgang der Schalteinrichtung (entsprechend dem Ausgang bei der Kontakt-

zunge 212 in Figur 3). In jeder Schalteinrichtung ist jeweils ein Schalter geschlossen. Welcher Schalter dies zu einem gegebenen Zeitpunkt ist, hängt von den an den Leitungen 212A, 222A...262A anliegenden Adressen-Bits ab, wobei jedem Schalter eine eindeutige Adresse zugeordnet ist. Die adressierbaren Schalter können beispielsweise kommerziell erhältliche CMOS-Schalter sein, wie beispielsweise der integrierte Schaltkreis CD4051CMOS von RCA. Aber auch jeder andere geeignete adressierbare oder programmierbare Schalterblock anderer Hersteller kann verwendet werden. Die Adressen für die einzelnen Schalteinrichtungen 211, 221...261 werden durch die zugeordneten Zähler 213, 223...263 erzeugt, die Taktzyklen (clock cycles) von zugeordneten Frequenzteilern 214, 224...264 zählen. Diese Frequenzteiler teilen die von einem Taktimpulsformer (clock) ausgehende Frequenz herunter. So wird beispielsweise bei jedem aufeinanderfolgenden Zyklus der vom Frequenzteiler 214 ausgehenden Signale die nächst höhere Zählstufe vom Zähler 213 erzeugt und daraus ergibt sich die nächst höhere Adresse für die adressierbaren Schalter der Schalteinrichtung 211. Die Schalter 300, 302...360 werden daher sequenziell von rechts nach links geschlossen, ebenso auch die Schalter der anderen Schalteinrichtungen 221, 231...261. Auf diese Weise erreicht man eine dynamische Fokussierung in der oben beschriebenen Art und Weise durch immer wieder wiederholtes Durchlaufen der Fokuspunkte des Darstellungssystems vom nahen Fokus bis zum fernen Fokus.

- 31 -

Da jede der Verzögerungsketten 210, 220...260 nach Figur 3 eine verschiedene Zahl von Stufen und Anzapfungen hat, hat jede der Schalteinrichtungen 211, 221...261 eine entsprechende verschiedene Zahl adressierbarer Schalter, d.h. die Schalteinrichtung 211 hat einundsechzig, die Schalteinrichtung 221 hat einundfünfzig Schalter und so weiter. (Die Zahl der Anzapfungen ist um eins größer als die Zahl der Stufen, da an beiden Enden jeder Verzögerungskette Anzapfungen sind.) Die Adressen für jede der Schalteinrichtungen werden durch die Frequenzteiler 214, 224...264 mit verschiedener Taktfolgegeschwindigkeit erzeugt. Speziell kann man die Taktfrequenz für jede einzelne Schalteinrichtung erhalten, indem man eine vom Taktimpulsformer 500 ausgehende Basistaktfrequenz mit Hilfe entsprechend dimensionierter Frequenzteiler herunter teilt. Dies kann z.B. folgendermaßen geschehen: Bezeichnet man die Durchlaufperiode (d.h. die Periode, während der alle Kontaktzungen nach Figur 3 oder alle Schalter nach Figur 4 einen vollen Durchlauf von rechts nach links absolvieren) als T, so ist jeder Stufe der Verzögerungsketten 210, 220...260 eine entsprechende Zeit T/60, T/50...T/10 zuzuordnen. Umgekehrt proportional dazu muß das Verhältnis der den Verzögerungsketten 210, 220...260 zugeordneten Taktfrequenzen 60:50:40:30:20:10 betragen. Daraus folgt, daß Frequenzen mit geeigneten Verhältnissen zu erhalten sind, wenn man mit einer Basistaktfrequenz von 600F beginnt und sie um einen Divisor 10, 12, 15, 20, 30 und 60 herunterteilt. So erhält man die gewünschten Frequenzen 60F, 50F, 40F, 30F, 20F und 10F. Diese Divisionen erhält man mit Hilfe der Frequenzteiler 214, 224...264.

Bei konstanter Basistaktfrequenz ist der Durchlauf der Fokuspunkte vom nahen Brennpunkt bis zum fernen Brennpunkt nicht linear, wie sich aus einer geometrischen Analyse des sich bewegenden Fokuspunktes ergibt. Demzufolge ist es vorteilhaft, die Frequenzen, aufgrund deren der synchronisierte Durchlauf der Schalter durch ihre zugeordneten Verzögerungsketten erfolgt, von einer variablen Basistaktfrequenz abzuleiten. Die variable Basisfrequenz wird von einem spannungskontrollierten Oszillator (VCO) 450 erzeugt, der seinerseits von einem vom Sägezahngenerator 425 erzeugten Sägezahnsignal kontrolliert wird. Auf ein Startsignal der Zeittaktschaltung 170 (Figur 2 - weiter oben beschrieben im Zusammenhang mit Figur 3) hin, erzeugt der Sägezahngenerator 425 ein abwärts laufendes Spannungssignal, das an den Kontrollanschluß des VCO 450 angeschlossen ist. Die Zeittaktschaltung 170 liefert auch das Einschaltsignal "e" an den Taktimpulsformer 500. Das Oszillatorausgangssignal beginnt dabei beispielsweise mit einer Anfangsfrequenz von 9,6 MHz und fällt, kontrolliert von dem Sägezahnsignal,ab bis zu einer Endfrequenz von beispielsweise 2,4 MHz. Dementsprechend verlangsamt sich der Durchlauf in den Schalteinrichtungen 211, 221...261, während der Fokuspunkt sich weiter weg bewegt. Nach Beendigung einer Abtastlinie werden die Zähler 213, 223...263 auf ein Signal der Zeittaktschaltung hin zurückgestellt. Dies ist in der Figur 4 durch den Buchstaben "r" für "reset" angedeutet. Wenn die Zähler auf Null zurückgestellt sind, sind die äußerst rechten Schalter (z.B. 300) geschlossen und die Schalteinrichtung ist bereit für den nächsten Zyklus von Zählsignalen der nach Anlegen des Einschaltsignals "e" den nächsten Durchlauf der Schalter durch die Verzögerungsketten steuert.

Aufgrund der digitalen Bauweise der bevorzugten Ausführungsform der Erfindung ist es vorteilhaft, die Anordnung der Ringe des Schallwandlers so zu wählen, daß gleich große zugeordnete Verzögerungsbeträge zwischen Ihnen zur Fokussierung verwendet werden können. Wie groß diese Verzögerungsbeträge sein müssen, hängt von der Geometrie ab. Sie können daher gleiche Zeitbeträge sein, wenn man die Abstände der Schallwandlerringe entsprechend wählt.

Während die vorgehend beschriebene bevorzugte Ausführungsform der Erfindung ein Verfahren zur dynamischen Fokussierung über einen Bereich von Entfernungen vom Schallwandler betrifft, kann man selbstverständlich die beschriebene Technik zur variablem Verzögerung auch für andere Zwecke verwenden, beispielsweise um einen Strahl zu steuern, indem man nebeneinander angeordnete, kreisförmige oder beliebig anders angeordnete Elemente benutzt und den Strahl steuert, indem man die jedem Element zugeordnete Verzögerung variiert. Dem Fachmann sind auch ohne weiteres Kombinationsmöglichkeiten von Strahlsteuerung und dynamischer Fokussierung zugänglich. Weiterhin kann man, indem man die Schalter nach Figur 4 einfach in einer bestimmten Stellung schließbar macht und eine geeignete Einrichtung vorsieht, um sie in dieser gewünschten

- 34 -

und zwischen den Verzögerungsketten koordinierten Position festzuhalten, aus der erfindungsgemäßen Einrichtung eine schaltbare Fokussierung erhalten, die vom Betriebspersonal auf jeden Fokuspunkt in ihren entsprechenden Tiefenbereich geschaltet werden kann. Beispielsweise kann diese Ausführungsform realisiert werden, indem man eine entsprechende Zahl von Taktimpulsen des Taktimpulsformers 500 einspeist und dann stoppt.

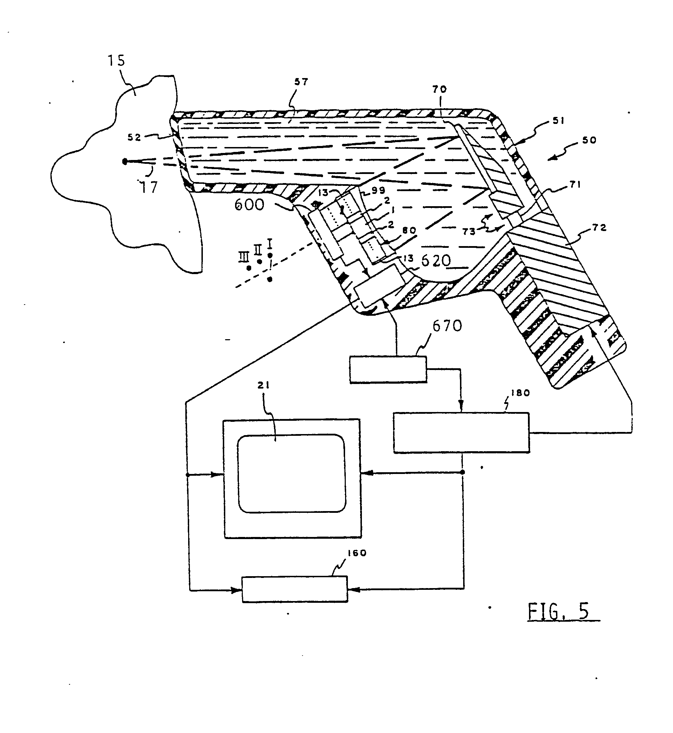

Figur 5 zeigt ähnlich wie Figur 1 eine Querschnittsdarstellung eines Teiles eines Abtastkopfes 50 verbunden mit einem Blockschaltbild, dient aber der Darstellung einer zweiten Hauptausführungsform der Erfindung. Bei der Ausführungsform nach Figur 5 sind die Elemente des Schallwandlers 80 an die Impulssender-/Empfängerschaltung 620 über eine neuartige Schaltung 600 zur wählbaren Fokussierung angeschlossen, die im folgenden beschrieben wird. Die Impulssender-/Empfängerschaltung ist außerdem an das Sichtgerät 21 und das Aufnahmegerät 160 angeschlossen und erhält Zeittaktsignale von der Zeittaktschaltung 670. Geeignete Schaltungsauslegungen für die Impulssender-/Empfängerschaltung und die Zeittaktschaltung sind in der Technik bekannt und nicht Gegenstand der vorliegenden Erfindung.

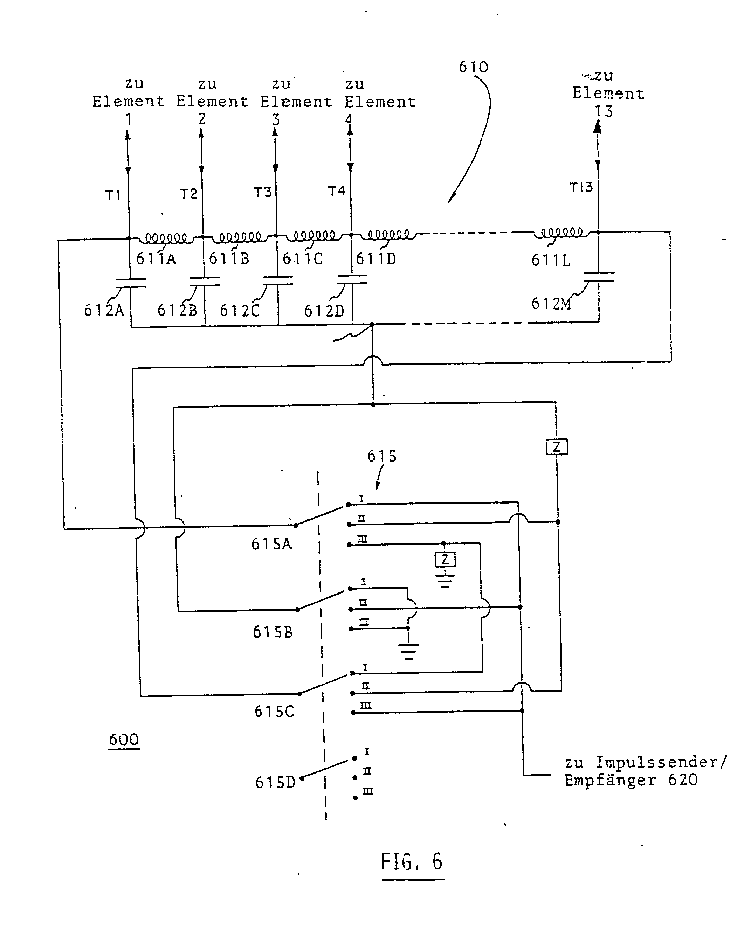

Figur 6 zeigt eine Ausführungsform der Schaltung zur wählbaren Fokussierung gemäß der zweiten Ausführungsform der Erfindung. Eine Verzögerungskette 610 schließt Verzögerungsstufen ein, die in konventioneller Weise eine seriell verbundene Folge (serially connected string) bilden. Bei der dargestellten Ausführungsform schließen die Verzögerungsstufen insgesamt zwölf Induktionen 611A, 611B...611L und insgesamt dreizehn Kapazitäten 612A, 612B...612M ein. Die Verzögerungskette 610 weist Anzapfungen T1, T2...T13 auf, die mit jeweils einer Platte der Kapazitäten 612A, 612B...612M verbunden sind. Die Induktionen 612A, 612B...612M sind zwischen jeweils benachbarten Anzapfungen geschaltet. Die jeweils andere Platte jeder Kapazität ist mit einem Anschluß 614 verbunden, den man üblicherweise als "gemeinsamen Pol" ("common connection") der Verzögerungskette bezeichnet. Die Anzapfungen T1, T2...T13 der Verzögerungsketten 610, die man allgemeiner auch als Verbindungseinrichtungen bezeichnen kann, sind außerdem jeweils mit geordneten Elementen 1, 2...13 des Schallwandlers 80 verbunden. Im Einzelnen ist die Anzapfung T1 mit dem Element 1, die Anzapfung T2 mit dem Element 2 und so weiter verbunden.

Die Schalteinrichtung 615 schließt drei Schalter 615A, 615B und 615C mit jeweils drei Schaltstellungen ein, die über eine gemeinsame Kontrolleinrichtung 615D betätigt werden. Die Kontaktzunge des Schalters 615A ist mit der Anzapfung T1 verbunden, die das eine Ende der Verzögerungskette 610 bildet. Die Kontaktzunge des Schalters 615C ist mit der Anzapfung T13 verbunden, die das andere Ende der Verzögerungskette 610 bildet. Weiterhin ist die Kontaktzunge des Schalters 615B mit dem gemeinsamen Pol 614 der Verzögerungskette 610 verbunden. In Position I des Schalters 615A, Position II des Schalters 615B und

- 36 -

Position III des Schalters 615C ist jeweils der Kontakt zu der Impulssender-/Empfängerschaltung 620 geschlossen (Figur 5). In Position III des Schalters 615A und Position I des Schalters 615C ist jeweils ein Kontakt nach Masse über eine charakteristische Impedanz Z hergestellt, die vorzugsweise der charakteristischen Impedanz der Verzögerungskette 610 entspricht. Die Positionen II der Schalter 615A und 615C liegen über eine Impedanz Z an dem gemeinsamen Pol 614 der Verzögerungskette an. Positionen I und III des Schalters 615B liegen an Masse.

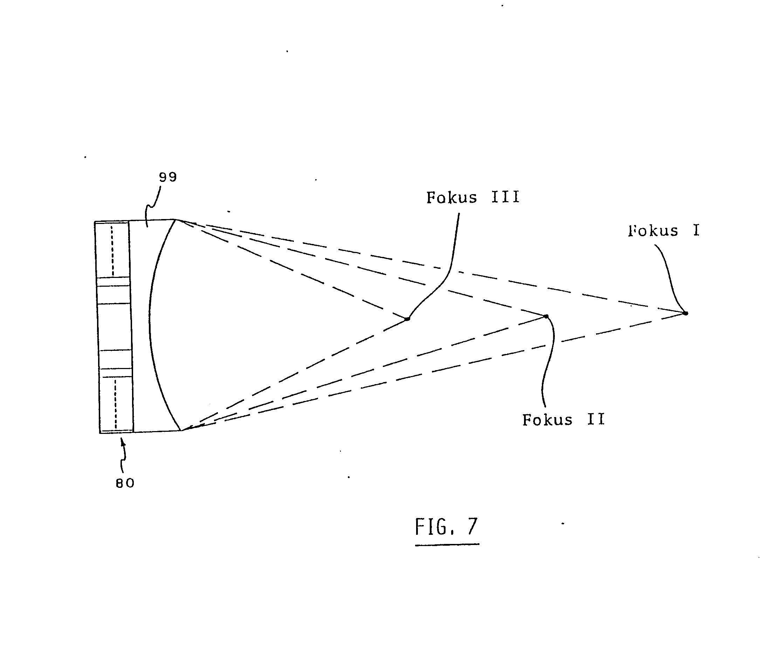

Die Vorrichtung arbeitet so, daß die drei Positionen I, II und III einem "fernen", einem "mittleren" (oder geometrischen) bzw. einem "nahen" Fokus zugeordnet sind. In der Schaltposition II schließt der Schalter 615B die Verbindung zwischen dem gemeinsamen Pol 614 der Verzögerungskette 610 und der Impulssender-/Empfängerschaltung 620 und die Schalter 615A und 615C sind jeweils über die Impedanz Z zum gemeinsamen Pol der Verzögerungskette geschlossen. Auf diese Weise ist die Impulssender-/Empfängerschaltung mit jedem der Ultraschallwandlerelemente verbunden, ohne eine relative Verzögerung zwischen den verschiedenen Elementen; und die Enden der Verzögerungskette 610 sind jeweils mit der charakteristischen Impedanz der Verzögerungskette abgeschlossen. Demzufolge ist in der Position II der Schalteinrichtung das System auf seinen geometrischen Brennpunkt fokussiert, der durch die Linse 99 (Figur 6) bestimmt wird. Dieser Brennpunkt wird in dem Diagramm der Figur 7 als "Fokus II" bezeichnet.

Befindet sich die Schalteinrichtung in Position I, so ist über den Schalter 615A das durch die Anzapfung T1 definierte Ende der Verzögerungskette 610 mit der Impulssender-/Empfängerschaltung verbunden. In dieser Position ist das durch die Anzapfung T13 definierte Ende der Verzögerungskette über dem Schalter 615B auf Massepotential geschaltet, während der gemeinsame Pol 614 der Verzögerungskette über dem Schalter 615C und die Impedanz Z an Massepotential anliegt. Folglich sind in dieser Schaltposition die den Wandlerelementen 1, 2...13 zugeordneten Verzögerungen immer größer für die Elemente mit höherer Nummer. Wandlerelement 13 ist nämlich mit der Impulssender-/Empfängerschaltung 620 über die gesamte Folge der Verzögerungsstufen verbunden, während das Wandlerelement 1 ohne zwischengeschaltete Verzögerungsstufen an die Impulssender-/Empfängerschaltung angeschlossen ist. Die dazwischen liegenden Elemente des Schallwandlers sind über eine zunehmende Anzahl von Verzögerungsstufen mit der Impulssender-/Empfängerschaltung verbunden, entsprechend wie ihre Nummer zunimmt. Das Resultat ist der in Figur 1 als "Fokus I" bezeichnete "ferne" Brennpunkt.

In der Schaltstellung III der Schalteinrichtung 615 ist die Situation gerade umgekehrt wie soeben beschrieben. In diesem Falle ist das der Anzapfung T13 zugeordnete Ende der Verzögerungskette 610 an die Impulssender-/Empfängerschaltung 620 über den Schalter 615C angeschlossen. Das andere, mit der Anzapfung T1 verbundene Ende der Verzögerungskette 620 liegt über den Schalter 615A und die charakteritstische Impedanz Z an Massepotential. Der gemeinsame Pol 614 der Verzögerungskette ist über Schalter 615B auf Masse geschaltet. In dieser Schaltstellung sind den Elementen mit kleiner werdender Nummer zunehmend größere Verzögerungen zugeordnet. Element 1 erfährt also die größte Verzögerung und Element 13 die geringste. Als Resultat ergibt sich ein Brennpunkt, der dem Schallwandler näherliegt, als der geometrische Fokus, nämlich der in der Figur 7 als "Fokus III" bezeichnete "nahe" Fokuspunkt.

Wenn das Betriebspersonal eine bestimmte Schalterstellung der Schalteinrichtung 615 mit Hilfe der Kontrolleinrichtung 615D gewählt hat, wird der ausgesandte Strahl unter Verwendung der ausgewählten Gruppe von Verzögerungen auf den gewählten Brennpunkt gelenkt. Die gleichen Verzögerungen werden auch beim Empfang benutzt. Selbstverständlich kann erfindungsgemäß aber auch ein System so gestaltet werden, daß die gewählte Fokussierung durch Verzögerung entweder nur beim Senden oder beim Empfangen des Ultraschallsignales benutzt wird, während im jeweils anderen Betriebszustand eine direkte Verbindung zwischen der Impulssender-/ Empfängerschaltung und dem Wandler verwendet wird. Die Kontrolleinrichtung 615D kann beispielsweise auch in dem Anzeige- und Kontrollgerät 20 untergebracht sein, während die Schalter 615A , 615B und 615C dann als Relais ausgeführt sind.

Weiterhin kann alternativ in der Schalterposition, die dem mittleren (oder geometrischen) Brennpunkt entspricht, das System so geschaltet sein, daß die Signale von allen Schallwandlerelementen zusammengefaßt werden und direkt an die Impulssender-/ Empfängerschaltung unter Umgehung der Verzögerungskette angelegt sind. Eine derartige Ausführungsform mag Vorteile haben, insoweit sie es vermeidet, daß die Signale über Komponenten der Verzögerungskette laufen. Andererseits ist damit ein Nachteil verbunden, da eine gewisse Zahl zusätzlicher Schalter (einer für jedes Wandlerelement) nötig wäre.

Dem Fachmann werden aufgrund der dargelegten bevor- zugten Version der zweiten Hauptausführungsform der Erfindung eine Vielzahl von Ausgestaltungsmöglichkeiten zugänglich sein, die den Grundgedanken der vorliegenden Erfindung benutzen. Beispielsweise muß nicht grundsätzlich eine Linse (oder eine andere zusätzliche Fokussierung, wie z.B. ein gekrümmter ·Schallwandler oder Festverzögerungen) benutzt werden. Selbstverständlich kann das System zur wählbaren Verzögerung nach der Erfindung auch für andere Zwecke benutzt werden, beispielsweise zum wählbaren Steuern eines Ultraschallstrahles auf verschiedene diskrete Positionen. Schließlich können selbstverständlich auch nur zwei der drei wählbaren Betriebsweisen mit verschiedener Verzögerung benutzt werden.

Bezugszeichen-Liste

| | |
|---|---|
| 1, 2 ... 13 | Elemente von 80 |
| 1A, 2A ... 13A | Verbindungsleitungen |
| 1B, 2B ... 13B | Vorverstärker |
| 15 | Körperteil |
| 17 | Begrenzung des Ultraschallstrahls |
| 21 | Sichtgerät |
| 48 | Kabel |
| 50 | Abtastkopf |
| 51 | Gehäuse |
| 52 | Abtastfenster |
| 70 | Abtastspiegel |
| 71 | Achse von 70 |
| 72 | Elektromotor |
| 73 | gekrümmter Doppelpfeil |
| 80 | Ultraschallwandler |
| 99 | Fokussierlinse |
| 120 | Impulserzeugungsschaltung |
| 160 | Aufnahmegerät |
| 170 | Zeittaktschaltung |
| 180 | Schaltung zur Kontrolle der Reflektorbewegung und der Sichtgerätablenkung |
| 200 | Schaltung zur dynamischen Fokussierung |
| 201 | Empfänger |
| 210, 220, 230 , 240, 250, 260 | Verzögerungsketten |
| 211, 221, 231, 241, 251, 261 | Schalteinrichtungen |
| 212, 222, 230, 242, 252, 262 | Kontaktzungen |
| 212A, 222A ... 262A | Kontrolleinrichtungen für 212, 222 ... 262 |
| 213, 223 ... 263 | Zähler |
| 281 - 286 | Summierungsschaltungen |
| 291 - 296 | Festverzögerungsschaltungen |
| 300, 302 ... 360 | Schalter |
| 425 | Sägezahngenerator |
| 450 | Spannungsgesteuerter Oszillator (VCO) |
| 500 | Taktimpulsformer |
| 600 | Schaltung zur wählbaren Fokussierung |

- 41 -                                                          0013029

| | |
|---|---|
| 610 | Verzögerungskette |
| 611A - 611L | Induktivitäten von 610 |
| 612A - 612L | Kapazitäten von 610 |
| 614 | gemeinsamer Pol von 610 |
| 615 | Schalteinrichtung |
| 615A, 615B, 615C | Schalter von 615 |
| 615D | Kontrolleinrichtung von 615 |
| 620 | Impulssender - Empfängerschaltung |
| 670 | Zeittaktschaltung |
| T1, T2 ... T13 | Abgriffe von 610 |

0013029

## Ansprüche

1. Verfahren zur variablen Verzögerung von von einer Mehrzahl von Elementen empfangenen Signalen und zur Summierung der Signale, wobei die einzelnen Beiträge der verschiedenen Elemente zum Summensignal gegeneinander verzögert sind und die relativen Verzögerungen zwischen den Signalen verschiedener Elemente sich zeitlich ändern, dadurch gekennzeichnet, daß die Signale von Paaren der Elemente an gegenüberliegende Enden von jeweils einer aus einer Mehrzahl von Verzögerungsketten angelegt werden, die Signale an verschiedenen Stufen der Verzögerungsketten durch diesen jeweils zugeordnete Schalteinrichtungen als Funktion der Zeit abgegriffen werden, so daß die Verzögerungen der von verschiedenen Elementen kommenden Signale sich als Funktion der Zeit ändern,und die Ausgangssignale der Schalteinrichtungen zusammengefaßt werden.

2. Verfahren nach Anspruch 1, dadurch gekennzeichnet, daß die Signale an aufeinanderfolgenden Stufen der Verzögerungsketten sequentiell abgegriffen werden.

3. Verfahren nach Anspruch 1 oder 2, dadurch gekennzeichnet, daß die Signale von den Stufen der Verzögerungsketten in zwischen den Verzögerungsketten synchronisierter Art und Weise abgegriffen werden.

4. Verfahren nach einem der Ansprüche 1 bis 3, dadurch gekennzeichnet, daß die Verzögerungsketten jeweils eine verschiedene Anzahl von Stufen haben, die Elemente geordnete Wandlerelemente sind und die Signale von dem ersten und letzten Element an entgegengesetzte Enden der Verzögerungskette mit der größten Stufenzahl angelegt werden, die Signale des zweiten und vorletzten Elements an entgegengesetzte Enden der Verzögerungskette mit der zweitgrößten Stufenzahl angelegt werden und so weiter.

5. Verfahren nach einem der Ansprüche 1 bis 4, dadurch gekennzeichnet, daß die Signale beim Zusammenfassen um feste Beträge dergestalt verzögert werden, daß die von einer Kette mit kleinerer Stufenzahl kommenden Signale jeweils stärker verzögert werden.

6. Verfahren nach einem der Ansprüche 3 bis 5, dadurch gekennzeichnet, daß die Synchronisationssignale für das Abgreifen von den Stufen der Verzögerungsketten durch Teilen einer Basisfrequenz in eine Mehrzahl von niedrigeren Frequenzen erzeugt werden.

7. Verfahren nach einem der Ansprüche 3 bis 6, dadurch gekennzeichnet, daß das Abgreifen der Signale von den Stufen der Verzögerungsketten mit Hilfe von den einzelnen Stufen jeder Verzögerungskette zugeordneten Schalteinrichtungen erfolgt und die Synchronisationssignale festlegen, welcher Schalter in jeder Verzögerungskette gerade geschlossen ist.

8. Vorrichtung zur Durchführung des Verfahrens nach einem der Ansprüche 1 bis 7, insbesondere zur Darstellung von Körperteilen, mit einer Sendeeinrichtung zum Einstrahlen von Energie in den Körper und einem Wandler zum Umwandeln von aus dem Körper reflektierten Echos in elektrische Signale, wobei der Wandler in eine Mehrzahl von abgegrenzten Elementen aufgeteilt ist und gleichzeitig Teil der Sendeeinrichtung sein kann, gekennzeichnet durch eine Mehrzahl von Verzögerungsketten (210, 220, 230, 240, 250, 260), Einrichtungen zum Anlegen der Signale von Paaren der Elemente (1, 13; 2, 12; 3, 11; 4, 10; 5, 9; 6, 8) an gegenüberliegende Enden jeweils einer der Verzögerungsketten, eine Mehrzahl von den Verzögerungsketten zugeordneten Schalteinrichtungen (211, 221, 231, 241, 251, 261) zum Abgreifen der Signale an verschiedenen Stufen der Verzögerungsketten und Kopplungseinrichtungen (281-286, 291-296) zur Zusammenfassung der Ausgänge der Schalteinrichtungen zur Formung eines Bildsignales.

9. Vorrichtung nach Anspruch 8, dadurch gekennzeichnet, daß die Verzögerungsketten (210, 220, 230, 240, 250, 260) jeweils eine verschiedene Anzahl von Stufen haben, die Elemente geordnete Wandlerelemente (1-13) sind und die Signale von dem ersten und letzten Element (1, 13) mit entgegengesetzten Enden der Verzögerungskette mit der größten Stufenzahl (210) verbunden sind, die Signale des zweiten und vorletzten Elementes (2, 12) mit entgegengesetzten Enden der Verzögerungskette mit der zweitgrößten Stufenzahl verbunden sind und so weiter.

10. Vorrichtung nach Anspruch 8 oder 9, dadurch gekennzeichnet, daß die Kopplungseinrichtungen Verzögerungseinrichtungen (291-296) einschließen, die so verdrahtet sind, daß nacheinander die Signale größeren festen Verzögerungen unterliegen, die von Verzögerungsleitungen mit nacheinander kleineren Anzahl von Stufen kommen.

11. Vorrichtung zum Verbinden mit variabler Verzögerung einer Vielzahl von Elementen mit einem einzelnen Element, gekennzeichnet durch eine Mehrzahl von Verzögerungsketten (210, 220, 230, 240, 250, 260), Einrichtungen zur Verbindung von Paaren der Elemente (1, 13; 2, 12; 3, 11; 4, 10; 5, 9; 6, 8) mit gegenüberliegenden Enden jeweils einer aus der Mehrzahl von Verzögerungsketten (210, 220, 230, 240, 250, 260), eine Mehrzahl von Schalteinrichtungen (211, 221, 231, 241, 251, 261), die jeweils einer der Verzögerungsketten zugeordnet und so verschaltet sind, daß eine bestimmte Verzögerungsstufe der zugehörigen Verzögerungskette mit dem einzelnen Element verbunden ist und Einrichtungen (212A, 222A, 232A, 242A, 252A, 262A) zum Wechseln der Verzögerungsstufen, an die die Schalteinrichtungen angeschlossen sind.

12. Vorrichtung nach Anspruch 11, dadurch gekennzeichnet, daß die Verzögerungsketten (210, 220, 230, 240, 250, 260) mit einer verschiedenen Anzahl von Verzögerungsstufen ausgestattet sind und die Schalteinrichtungen (211, 221, 231, 241, 251, 261) mit dem einzelnen Element über Verzögerungseinrichtungen so verbunden sind, daß nacheinander größere Verzögerungen zwischen dem einzelnen Element und den Verzögerungsketten auftreten in entsprechender Weise, wie die Anzahl der Stufen der Verzögerungsketten abnimmt.

13. Vorrichtung nach einem der Ansprüche 11 oder 12, dadurch gekennzeichnet, daß die Einrichtungen (212A, 222A...262A) zum Wechseln der gewählten Verzögerungsstufe, an die die Schalteinrichtungen (21,, 221...261) angeschlossen sind, so betrieben werden, daß aufeinanderfolgende Verzögerungsstufen nacheinander angeschlossen werden.

14. Vorrichtung mit schaltbarer Verzögerung zum Empfang von von einer Mehrzahl von Elementen ausgehenden Signalen und zur Erzeugung eines Kombinationssignales aus den Signalen, wobei die Beiträge von verschiedenen Elementen zu dem Kombinationssignal gegeneinander verzögert und die den Signalen von den verschiedenen Elementen zugeordneten relativen Verzögerungen durch eine Betriebsperson schaltbar sind, gekennzeichnet durch eine Mehrzahl von Verzögerungsketten (210, 220, 230, 240, 250, 260), Einrichtungen zum Anlegen der Signale von Paaren der Elemente (1, 13; 2, 12; 3, 11; 4, 10; 5, 9; 6, 8) an gegenüberliegende Enden jeweils einer der Verzögerungsketten, eine Schaltung (281-286, 291-296) zur Kombination der Signale und eine Mehrzahl von der Mehrzahl der Verzögerungsketten jeweils zugeordneten Schalteinrichtungen (211, 221...261), die jeweils so betrieben werden, daß das an einer vom Betriebspersonal gewählten Verzögerungsstufe anliegende Signal an die Kombinationsschaltung angelegt ist.

15. Vorrichtung nach Anspruch 14, dadurch gekennzeichnet, daß die Verzögerungsketten (210, 220, 230, 240, 250, 260) jeweils eine verschiedene Anzahl von Stufen haben, die Elemente geordnete Wandlerelemente (1-13) sind und die Signale von dem ersten und letzten Element
(1,13) mit entgegengesetzten Enden der Verzögerungskette mit der größten Stufenzahl (210) verbunden sind,
die Signale des zweiten und vorletzten Elementes (2, 12)
mit entgegengesetzten Enden der Verzögerungskette mit
der zweitgrößten Stufenzahl verbunden sind und so
weiter.

16. Vorrichtung zum Verbinden mit wählbarer Verzögerung
von einer Mehrzahl von Elementen mit einem Eingangs-/
Ausgangsanschluß, bei der die relativen Verzögerungen
zwischen dem Eingangs-/Ausgangsanschluß und den einzelnen Elementen vom Bedienungspersonal wählbar sind,
gekennzeichnet durch eine Verzögerungskette (610)
mit mehreren festen Verzögerungsstufen in einer
seriell verbundenen direkt gekoppelten Folge, Verbindungseinrichtung (T 1 - T 13) zum Verbinden der
Verzögerungsstufen mit den zugeordneten Elementen
(1 - 13) und Schalteinrichtungen (615A, 615B, 615C,
615D) zum vom Bedienungspersonal wählbaren Verbinden
des einen oder anderen Endes der Verzögerungskette
mit dem Eingangs-/Ausgangsanschluß.

17. Vorrichtung nach Anspruch 16, dadurch gekennzeichnet,
daß die Schalteinrichtung (615A, 615B, 615C, 615D)
Mittel zur Kopplung jedes der Elemente (1-13) ohne
relative Verzögerung zueinander mit dem Eingangs-/
Ausgangsanschluß einschließt.

18. Vorrichtung nach Anspruch 17, dadurch gekennzeichnet,
daß die Mittel zur relativverzögerungsfreien Kopplung
der Elemente eine Verbindung zwischen dem gemeinsamen
Pol der Verzögerungskette (614) und dem Eingangs-/
Ausgangsanschluß einschließen.

19. Vorrichtung nach einem der Ansprüche 16 - 18, dadurch gekennzeichnet, daß die Schalteinrichtung (615A, 615B, 615C, 615D) Mittel einschließt, um eine Abschlußimpedanz an das mit dem Eingangs-/Ausgangsanschluß nicht verbundene Ende der Verzögerungskette (610) zu schalten.

20. Vorrichtung zur bildlichen Darstellung eines Körperteils mit einem Impulssender/Empfänger, einem Schallwandler zur Einstrahlung von Ultraschallenergie in den Körper und zur Umwandlung von aus dem Körper reflektiertem Ultraschall in elektrische Signale und einer an den Impulssender/Empfänger angeschlossenen Einrichtung zur bildlichen Darstellung des Körperteils, gekennzeichnet durch eine Verzögerungskette (610) mit mehreren festen Verzögerungsstufen in einer seriell verbundenen direkt gekoppelten Folge, Verbindungseinrichtung (T1 - T13) zum Verbinden der Verzögerungsstufen mit den zugeordneten Elementen (1-13) und Schalteinrichtungen (615A, 615B, 615C, 615D) zum vom Bedienungspersonal wählbaren Verbinden des einen oder anderen Endes der Verzögerungskette mit dem Eingangs-/Ausgangsanschluß.

21. Vorrichtung nach Anspruch 20, dadurch gekennzeichnet, daß die Schalteinrichtung (615A, 615B, 615C, 615D) Mittel zur Kopplung jedes der Elemente (1-13) ohne relative Verzögerung zueinander mit dem Eingangs-/Ausgangsanschluß einschließt.

22. Vorrichtung nach Anspruch 21, dadurch gekennzeichnet, daß die Mittel zur relativverzögerungsfreien Kopplung der Elemente eine Verbindung zwischen dem gemeinsamen Pol der Verzögerungskette (614) und dem Eingangs-/Ausgangsanschluß einschließen.

0013029

23. Vorrichtung nach einem der Ansprüche 20 bis 22, dadurch gekennzeichnet, daß die Schalteinrichtung (615A, 615B, 615C, 615D) Mittel einschließt, um eine Abschlußimpedanz an das mit dem Eingangs-/Ausgangsanschluß nicht verbundene Ende der Verzögerungskette (610) zu schalten.

$- \frac{1}{6} -$

0013029

Fig. 1

Fig. 2

Ausgang

Fig. 3

Fig. 4

FIG. 5

FIG. 6

99

Fokus III

Fokus I

Fokus II

80

FIG. 7

- 9/9 -

0013029

0013029

Nummer der Anmeldung

EP 79 10 5389

Europäisches Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 2 406 340 (BATCHELDER)<br>* Insgesamt *<br><br>-- | 1,2,11,<br>13,14 |
| | US - A - 4 084 582 (NIGAM)<br>* Titelblatt: Zusammenfassung; Spalte 2, Zeile 50 - Spalte 3, Zeile 22; Spalte 4, Zeilen 52-61; Spalte 5, Zeilen 13-57; Spalte 6, Zeilen 28-57; Figuren 1-3 *<br><br>-- | 1,8,<br>11,14 |
| | US - A - 4 005 382 (BEAVER)<br>* Titelblatt: Zusammenfassung; Figuren 4,5; Spalte 1, Zeilen 6-15; Spalte 4, Zeilen 29-67; Spalte 5, Zeile 18 - Spalte 8, Zeile 16; Spalte 9, Zeilen 38-63 *<br><br>-- | 16,17,<br>20,21 |
| | DE - A - 2 736 310 (HEWLETT-PACKARD)<br>* Ansprüche; Seite 3, Zeile 29 - Seite 5, Zeile 26; Figur *<br>& US - A - 4 116 229<br><br>-- | 16,20 |
| P | GB - A - 2 017 302 (NEW YORK INSTITUTE OF TECHNOLOGY)<br>* Titelblatt: Zusammenfassung; Figuren 3,7; Seite 1, Zeilen 3-8; Seite 4, Zeilen 43-47; Seite 4, Zeile 102 - Seite 5, Zeile 5; Seite 6, Zeilen 19-49 * | 1,8,<br>11,14 |
| D | & DE - A - 2 911 613<br><br>---- | |

### KLASSIFIKATION DER ANMELDUNG (Int.Cl.)

G 01 S 15/89
G 10 K 11/34
A 61 B 10/00

### RECHERCHIERTE SACHGEBIETE (Int. Cl.)

G 01 S   3/82
         7/62
        15/89
G 01 N  29/00
        29/04
G 01 H   3/12
G 10 K  11/34
A 61 B  10/00

### KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 31-03-1980 | OLDROYD |

EPA form 1503.1   06.78